# EUROPEAN PATENT SPECIFICATION

(11) **EP 0 441 384 B1**
(45) Date of publication and mention of the grant of the patent: **01.12.1999**
(21) Application number: 91101706.9
(22) Date of filing: 07.02.1991
(51) Int. Cl.: A61M 29/02, A61M 25/01

(54) **Readily exchangeable perfusion catheter**
Leicht auswechselbarer Perfusionskatheter
Cathéter de perfusion facilement interchangeable

(30) Priority: 07.02.1990 US 476056
(43) Date of publication of application: 14.08.1991
(73) Proprietor: ADVANCED CARDIOVASCULAR SYSTEMS, INC., Santa Clara California 95052-8167 (US)
(72) Inventor: Peter R. McInnes, Camberley, Surrey GU 179 JL (GB)
(74) Representative: Baillie, Iain Cameron

(56) References cited:
- EP-A- 0 394 969
- US-A- 4 748 982
- US-A- 4 790 315
- US-A- 4 877 031
- US-A- 4 892 519

## Description

This invention generally relates to a dilatation catheter for angioplasty procedures such as percutaneous transluminal coronary angioplasty (PTCA).

In PTCA procedures, a dilatation catheter having an inflatable, relatively inelastic balloon on the distal end thereof is advanced through a patient's arterial system until the balloon crosses the atherosclerotic lesion to be dilated. The balloon is inflated to a predetermined size with radiopaque liquid at relatively high pressures (e.g., 8 atmospheres) to dilate the stenotic region and then the balloon is deflated so that the catheter can be removed and blood flow resumed.

Usually a guiding catheter having a preformed distal end is first percutaneously introduced into the patient's arterial system and advanced therein until the distal tip of the guiding catheter is disposed in the appropriate ostium of the patient's coronary artery. A guidewire is preloaded within a dilatation catheter and both are advanced through the previously positioned guiding catheter to the distal end thereof. The guidewire is first advanced out of the guiding catheter into the patient's coronary anatomy until the distal end of the guidewire crosses the stenotic region to be dilated. The dilatation catheter is then advanced over the guidewire, with the guidewire slidably disposed within an inner lumen of the catheter until the inflatable balloon is positioned within the stenosis. The balloon is inflated to a relatively high pressure to dilate the stenosis and then deflated and removed over the guidewire. For a detailed description of procedures, reference is made to U.S. Patent No. 4,332,254 (Lundquist), U.S. Patent 4,323,071 (Simpson-Robert), U.S. 4,439,185 (Lundquist), U.S. Patent 4,468,224 (Enzmann et al.), U.S. 4,516,972 (Samson), U.S. Patent 4,538,622 (Samson et al.), U.S. 4,554,929 (Samson et al.), U.S. Patent 4,569,347 (Frisbie), U.S. Patent 4,571,240 (Samson et al.), U.S. Patent 4,638,805 (Powell), U.S. Patent 4,748,982 (Horzewski et al.).

Efforts have been made to develop dilatation catheters which perfuse blood through an inner lumen of the catheter which traverses the interior of the balloon when the balloon is inflated during angioplasty procedures in order to avoid ischemic conditions distal to the inflated balloon. For example, dilatation catheters providing perfusion capabilities are described in U.S. Patent 4,423,725 (Baran et al.) and U.S. Patent 4,790,315 (Mueller, Jr. et al.). See also, U.S. Patent 4,581,017 (Sahota). However, these perfusion dilatation catheters generally have relatively large deflated profiles and as a result they frequently are not employed in those situations where the stenoses to be treated are deep within the patient's coronary anatomy.

Additionally, in instances where there is an acute or sudden blockage of the arterial passageway after dilatation of a stenotic region, conventional dilatation non-perfusion type catheters must first be removed from the patient before a perfusion-type dilatation catheter can be advanced over the guidewire in place within the patient. Usually, such catheter exchanges require the use of an exchange wire or extension wire such as described in U. S. Patent 4,827,941 (Taylor et al.), which can add considerable time and complexity to a procedure frequently performed under emergency conditions.

In U.S. Patent Number 4,748,982 (Horzewski et al.), there is disclosed a balloon dilatation catheter adapted to be utilized with a guidewire and guiding catheter. The catheter, which diminishes in diameter along its length, includes a balloon on its distal section and a sleeve defining a guidewire lumen therein. The sleeve has a slit to permit the removal of the guidewire. U.S. Patent Number 4,877,031 (Conway and McInnes) discloses a steerable perfusion dilatation catheter. The device provides for the perfusion of blood when the balloon is inflated. The guidewire extends through an end torquing knob, inside the catheter body to the distal end of the catheter. U.S. Patent Number 4,790,315 (Mueller, Jr. et. al) discloses a perfusion dilatation catheter which resembles that of the present invention. However, the body of the earlier catheter is of a constant diameter with perfusion ports therein and has a guidewire extending along its length from a three-arm adapter.

What has been needed and heretofore unavailable is a perfusion-type dilatation catheter which can quickly and easily be introduced into a patient's arterial system and which has sufficient pushability to be advanced deep within the patient's vasculature. The present invention satisfies this need.

### SUMMARY OF THE INVENTION

The present invention provides a vascular catheter having a body having distal and proximal ends and an expandable member on the distal end; an inner lumen; a proximal and a distal guidewire port; and perfusion ports in the wall of the catheter body, the perfusion ports being in fluid communication with the inner lumen between the proximal guidewire port and the distal guidewire port in the distal end of the catheter, the guidewire port being disposed proximal to both the expandable member and the proximal perfusion ports and distal to the proximal end of the catheter, the distal end of the catheter body defining a first portion proximal to the expandable member which has perfusion ports and a second portion proximal to the first portion distal to the proximal guidewire port and which does not have perfusion ports, the catheter being advanceable over a guidewire slidably disposed within the inner lumen, the expandable member on the catheter being expandable to at least partially occlude a blood vessel at the location causing blood to flow through the proximal perfusion ports and the inner lumen and out of the distal perfusion ports, the expandable member being contractible to facilitate removal of the catheter from the patient, the catheter being characterized by the first portion having an outer maximum transverse dimension that is larger than the outer maximum transverse dimension of the second portion; the inner lumen extending in the distal portion of the catheter body between the distal guidewire port and the proximal guidewire port and 10 to 50 cm proximal from the distal guidewire port.

The present invention is directed to a dilatation catheter which can be readily exchanged without the need for extension wires or for the replacement of the guidewire with an exchange wire and which can also perfuse blood distal to the catheter when a vascular procedure is being performed within the blood vessel which otherwise blocks the flow of blood through.

A catheter in accordance with a preferred embodiment of the invention has an elongated catheter body with an inflatable, relatively inelastic balloon near the distal end thereof. The catheter body has a first elongated inner lumen extending from the proximal end of the catheter body to the interior of the inflated balloon near the distal end thereof to deliver inflation fluid to the interior of the balloon. A second, much shorter inner lumen extends within the distal portion of the catheter body between a proximal guidewire port and a distal guidewire port provided in the distal end of the catheter body. The distal guidewire port is in the very distal tip of the catheter body and the proximal guidewire port is at least 10 cm but not more than about 50 cm from the distal guidewire port. The second, much shorter lumen within the catheter body is adapted to slidably receive a guidewire to facilitate the advancement of the catheter over the guidewire into the patient's coronary anatomy.

At least one proximal perfusion port is provided in the catheter body between the proximal guidewire port and the proximal end of the balloon and at least one distal perfusion port is provided in the catheter body between the distal end of the balloon and the distal end of the catheter body. Both the proximal and distal perfusion ports are in fluid communication with the second smaller lumen disposed within the catheter body so that blood flows distal to the catheter when the balloon is inflated during the vascular procedure. The number, size and location of the perfusion ports can be varied depending upon the blood flow required, the size of the catheter and the size of the inner lumen. Typically, there may be 6 to 20 perfusion ports proximal to the balloon and about 4 to 12 perfusion ports distal to the balloon. In a preferred embodiment 10 ports are provided proximal to the balloon and 8 are provided distal to the balloon.

The proximal end of the catheter body is provided with an adapter with at least one arm for the delivery of inflation fluid from a high pressure source such as a syringe to the first inner lumen leading to the interior of the balloon for inflation purposes.

Preferably, the catheter wall which defines at least in part the second, smaller lumen disposed within the catheter body is provided with a slit which extends from the proximal guidewire port to a location proximal to the section containing the proximal perfusion ports. The purpose of this slit, as described in U.S. Patent 4,748,982 (Horzewski et al.) allows the guidewire to be pulled out of a significant portion of the second lumen to increase the ease in which catheters can be exchanged.

The portion of the elongated catheter body proximal to the proximal guidewire port is provided with a stiffening member such as a rod or wire which increases the pushability of the catheter and thereby allows for more distal advancement of the catheter into the patient's coronary anatomy than previous perfusion-type catheters.

In the performance of an angioplasty procedure utilizing the catheter assembly of the invention, it is preferred to preload the guidewire within the second smaller lumen of the catheter with the distal tip of the guidewire extending out of the distal tip of the catheter, and then advance the combined assembly through a guiding catheter previously disposed within the patient's vasculature with the distal tip of the guiding catheter disposed with the ostium of the patient's coronary artery. The guidewire is first extended out of the distal end of the guiding catheter into the patient's coronary artery until the distal tip of the guidewire crosses the stenotic region to be dilated. The dilatation catheter is then advanced out of the guiding catheter over the guidewire until the balloon on the dilatation catheter is positioned across the stenosis. The balloon is then inflated with the radiopaque liquid as conventionally practiced to dilate the stenosis.

An alternate procedure which has been found suitable comprises first advancing the guidewire through the guiding catheter and into the desired location within the patient's coronary anatomy and then mounting the dilatation catheter of the invention on the proximal end of the guidewire and advancing the catheter over the wire to the desired location within the patient's coronary arteries.

When the balloon is inflated, it occludes the artery and blocks normal blood flow therethrough. However, blood flows through the proximal perfusion ports, through the smaller second lumen, and then out the distal perfusion ports and the distal guidewire port located in the catheter body distal to the balloon. To maximize blood flow through the second lumen, it is preferred to withdraw the guidewire sufficiently from the dilatation catheter so that the distal portion of the guidewire remains in the second lumen but proximal to the portion of the second lumen between the proximal and distal perfusion ports. When the dilatation has been completed, the guidewire can then be advanced back through the second lumen and out the distal end thereof so that it crosses the stenosis.

In the event of an abrupt reclosure when the dilatation catheter is deflated, such as from a dissected lining, the balloon can be inflated in the stenotic region so as to maintain the patency of the artery. The artery may then be held open while blood perfuses therethrough for a long enough period to allow the dissected lining to be resecured to the blood vessel wall by natural healing or to allow for surgical procedures to be initiated to correct the abrupt reclosure, such as bypass surgery.

Should the catheter in place need to be replaced with another catheter, for example when the inflated diameter of the balloon on the catheter in place is too small to completely dilate a stenosis, a second catheter should then be inserted to complete the dilation. In this instance, the catheter of the invention can be readily replaced by holding onto the guidewire extending out the proximal end of the guiding catheter and pulling on the dilatation catheter to remove it from the patient. A second dilatation catheter of essentially the same construction but with a larger diameter balloon may then be mounted on the proximal end of the guidewire and then advanced over the guidewire into the stenosis for further dilation.

A similar situation arises when a second stenosis distal to the first stenosis needs to be dilated and the balloon on the catheter used to dilate the first stenosis is too large for the distal region. The same procedures may be followed to advance a catheter having a smaller balloon to the more distal stenosis.

The dilatation catheter in accordance with the present invention can be advanced deeply within the patient's vascular system, much further than prior perfusion catheters due to the increased pushability of the catheter. Thus, the catheter of the present invention allows for the long-term dilatation of stenoses which the prior perfusion catheters were unable to reach. Additionally, when a catheter in accordance with the present invention needs to be replaced with another catheter, such catheter exchanges can be quickly and very easily performed without the need for exchange wires or extension wires required with the prior art dilatation catheters. These and other advantages of the present invention will become more apparent from the following detailed description thereof when taken in conjunction with the attached exemplary drawings.

### BRIEF DESCRIPTION OF THE DRAWINGS

FIGURE 1 is an elevational view partially in section of a dilatation catheter embodying features of the invention;
FIGURE 2 is a transverse cross-sectional view taken along the lines 2-2 shown in FIGURE 1;
FIGURE 3 is a transverse cross-sectional view taken along the lines 3-3 shown in FIGURE 1;
FIGURE 4 is a transverse cross-sectional view taken along the lines 4-4 shown in FIGURE 1;
FIGURE 5 is a transverse cross-sectional view taken along the lines 5-5 shown in FIGURE 1;
FIGURE 6 is a transverse cross-sectional view taken along the lines 6-6 shown in FIGURE 1; and
FIGURE 7 is a longitudinal, center line, cross-sectional view taken through the transition region of the catheter shown in FIGURE 1 illustrating the extension of the guidewire through a proximal guidewire port and into an inner lumen of the dilatation catheter.

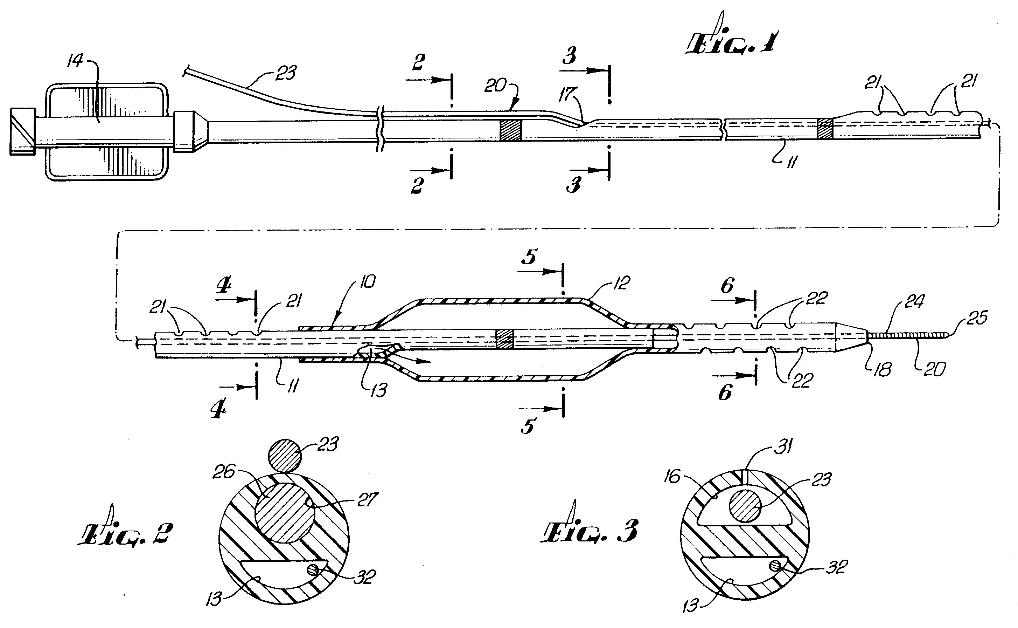

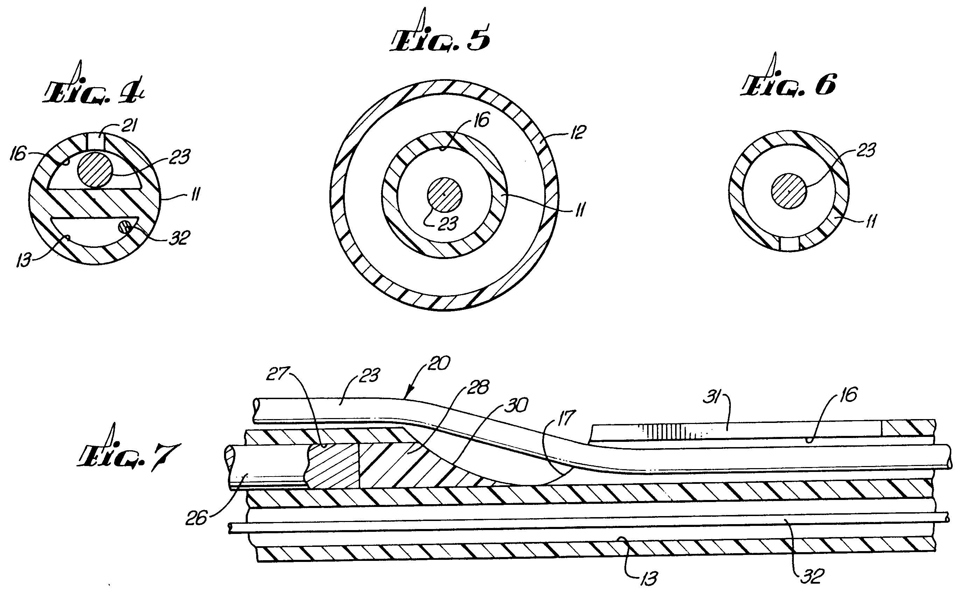

### DETAILED DESCRIPTION OF THE INVENTION

In accordance with a preferred embodiment, the present invention provides a vascular catheter 10 having a elongated catheter body 11 with an inflatable balloon 12 near the distal end thereof. A first inner lumen 13 extends through a substantial portion of the catheter body 11 and is in fluid communication with the interior of the balloon 12. An adapter 14 is provided at the proximal end of the catheter body 11 which is in fluid communication with the first inner lumen 13 to direct inflation fluid from a high pressure source such as a syringe pump (not shown) to the interior of balloon 12.

A second lumen 16 is provided in a distal portion of the catheter 10 which remains within the patient during angioplasty or other vascular procedures. The second lumen 16 is much shorter than the first lumen and extends between a proximal guidewire port 17 and a distal guidewire port 18 which is located at the distal tip of the catheter body. The proximal guidewire port 17 is located 10 to 50 cm, preferably 12 to 40 cm, from the distal guidewire port 18. During the angioplasty procedures, the guidewire 20 is slidably disposed within the second inner lumen 16.

Proximal perfusion ports 21 are provided in the catheter body 11 between the proximal end of the balloon 12 and the proximal guidewire port 17 and distal perfusion ports 22 are provided between the distal end of the balloon and the distal end of the catheter body 11. Perfusion ports 21 and 22 pass through the wall of the catheter body 11 which defines at least in part the second inner lumen 16 and therefore are in fluid communication therewith.

The guidewire 20 generally includes a core member 23 and a flexible body such as a helical coil 24 on the distal portion of the core member. A rounded plug 25 is provided at the distal tip of the core to prevent traumatic engagement with the arterial lining. During angioplasty or other vascular procedures, the proximal guidewire port 17 remains within the guiding catheter, and the core member 23 of the guidewire 20 extends out of the proximal guidewire port and runs generally parallel to the catheter body within the guiding catheter (not shown).

Stiffening rod 26 is disposed within a third lumen 27 provided in the catheter body 11 proximal to the proximal guidewire port 17 and generally extends to the proximal end of the catheter body 11. For ease of manufacturing, the third lumen 27 and the second inner lumen 16 are essentially the same lumen with a plug 28 provided therein proximally adjacent the proximal guidewire port 17. Preferably the distal portion of the plug 28 is in the form of a ramp 30 which can guide the guidewire 20 into or out of the second inner lumen 16. The wall of the catheter body 11 defining the inner lumen 16 is provided with a slit 31 from the proximal guidwire port 17 to a location proximal to the proximal perfusion port 21 through port 17.

The first inner lumen 13 is preferably provided with a small diameter wire member 32 which prevents the retention of air bubbles at the corners of the D-shaped first lumen. The wire member 32 preferably does not extend along essentially the entire length of the inflation lumen 13.

The various components of the catheter can be made from conventional materials. Catheter body 11 can be extruded or otherwise formed from plastic resins such as polyethylene and polyesters (e.g., Hytrel) and the balloon can be formed from polyethylene or polyethylene terephthalate resins. The core 23 of the guidewire 20 can be made of stainless steel and the coil 24 can be made of a more highly radiopaque material such as platinum, tungsten, palladium, ruthenium, rheniun and alloys thereof. A wide variety of other suitable materials can also be used for these components.

For coronary angioplasty procedures, the outer diameter of the catheter body 11 proximal to the perfusion section can typically range from about 0.035 to about 0.05 inch (0.89-1.30 mm.) and is smaller than the outer diameter of the perfusion section thereof which can range from about 0.04 to 0.06 inch (1.02-1.52 mm.). Inflatable balloon diameters can range from about 1.5 to about 4.5 mm. The stiffening element is a rod or wire preferably with a circular transverse cross-section ranging in diameter from about 0.015 to about 0.025 inch (0.38-0.64 mm.). The diameter of the guidewire lumen 16 in the perfusion section of the catheter body 11 may vary from about 0.015 to about 0.045 inch (0.38-1.14 mm.), but in the distal tip of the catheter it may range from about 0.015 to about 0.025 inch (0.38-0.64 mm). The overall length of the catheter body 11 from the distal tip to the adapter 14 may be about 130 to about 150 cm. The aforesaid dimensions are believed to be suitable for most coronary angioplasty procedures. Angioplasty procedures at other locations and catheters for other procedures (e.g., atherectomy procedures) may require dimensions different than those described above.

While the present invention has been described herein in terms of certain specifically preferred embodiments specifically directed to coronary angioplasty procedures, various modifications and improvements can be made without departing from the scope of the invention as defined in the claims annexed hereto.

## Claims

1. A vascular catheter (10) having a body (11) having distal and proximal ends and an expandable member (12) on the distal end; an inner lumen (16); a proximal and a distal guidewire port (17,18); and perfusion ports (21,22) in the wall of the catheter body (11), the perfusion ports (21,22) being in fluid communication with the inner lumen (16) between the proximal guidewire port (17) and the distal guidewire port (18) in the distal end of the catheter (10), the guidewire port (17) being disposed proximal to both the expandable member (12) and the proximal perfusion ports (21) and distal to the proximal end of the catheter, the distal end of the catheter body (11) defining a first portion proximal to the expandable member (12) which has perfusion ports and a second portion proximal to the first portion distal to the proximal guidewire port (17) and which does not have perfusion ports, the catheter (10) being advanceable over a guidewire (23) slidably disposed within the inner lumen (16), the expandable member (12) on the catheter (10) being expandable to at least partially occlude a blood vessel at the location causing blood to flow through the proximal perfusion ports (21) and the inner lumen (16) and out of the distal perfusion ports (22), the expandable member (12) being contractible to facilitate removal of the catheter (10) from the patient, the catheter being characterized by:
the first portion having an outer maximum transverse dimension that is larger than the outer maximum transverse dimension of the second portion;
the inner lumen (16) extending in the distal portion of the catheter body (11) between the distal guidewire port (18) and the proximal guidewire port (17) and 10 to 50 cm proximal from the distal guidewire port (18).

2. The catheter of the preceding claim 1 further characterized by:
the proximal perfusion ports (21) extending along a length of the first portion of the distal end of the catheter body (11);
means (26,27) to stiffen a portion of the catheter body (11) proximal to the proximal guidewire port (17) to provide the catheter (10) with improved pushability;
the proximal perfusion ports (21) being located between the proximal guidewire port (17) provided in the catheter body (11) and the expandable member (12);
the distal perfusion ports (22) provided in the catheter body (11) being located between the distal guidewire port (18) and the distal end of the expandable member (12);
the proximal guidewire port (17) being disposed at least 10 cm but not more than 50 cm from the distal end of the catheter (10), such that the catheter (10) is a readily exchangeable dilatation catheter (10).

3. The catheter (10) of claim 2 further characterized in that the means (26, 27) to stiffen the catheter body (11), is located proximal to the proximal guidewire port (17) and is a rod (26) tightly fitted within an inner lumen (27) of the catheter body extending from the proximal end of the catheter body (11) to a location proximal to the proximal guidewire port (17).

4. The catheter (10) of any one of the preceding claims further characterized in that the expandable member (12) is an inflatable, relatively inelastic balloon (12) suitable for dilating a stenosis, the balloon (12) being formed from a plastic resin selected from the group consisting of polyethylene and polyethylene teraphthalate.

5. The catheter (10) of any one of the preceding claims further characterized in that the catheter (10) has an inflation lumen (13) which extends distally from the proximal end of the catheter body (11) to the interior of the balloon (12), the proximal guidewire port (17) being disposed about 12 to about 40 cm from the distal guidewire port (18).

6. The catheter (10) of any one of the preceding claims further characterized in that the inflation lumen (13) has a D-shaped transverse cross section and a thin wire (32) extends within the D-shaped inflation lumen (13) to prevent retention of air bubbles in a corner of the inflation lumen.

7. The catheter (10) of any one of the preceding claims further characterized by a slit (31) provided in the wall of the catheter body (11) defining at least in part the inner lumen (16) which extends from the proximal guidewire port (17) to a location proximal to the proximal perfusion ports (21).

8. The catheter (10) of claim 6 further characterized in that there are about 6 to about 20 proximal perfusion ports (21) and about 4 to about 12 distal perfusion ports (22) provided in the catheter wall.

9. The catheter (10) of any one of the preceding claims further characterized by the guidewire (20, 23) and catheter (10) being dimensioned such as to enable one to at least partially remove the guidewire (20, 23) from the portion of the inner lumen (16) between the proximal (17) and distal (18) perfusion ports.

10. The catheter (10) of any one of the preceding claims further characterized by a second dilatation catheter for replacing or using as a further catheter to the catheter (10), the second dilatation catheter also having an expandable member (12) on the distal end thereof and an inner lumen (16) therein extending in a distal portion of the catheter body between a distal guidewire port and a proximal guidewire port about 10 to about 50 cm proximal from the distal guidewire port and perfusion ports in the wall of the catheter body in fluid communication with the inner lumen (16) between the proximal guidewire port and the distal guidewire port in the distal end of the catheter body; wherein the second dilatation catheter is mounted onto the guidewire (20, 23) by passing the proximal end of the guidewire (20, 23) through the lumen until a portion thereof extends out of the proximal guidewire port; holding the portion of the guidewire (20, 23) extending out of the proximal port of the catheter; and advancing the catheter over the guidewire (20, 23) into and through the vascular system of the patient until the catheter is positioned at a desired location therein .

11. The catheter (10) of any one of the preceding claims further characterized by the guidewire (20, 23) having proximal and distal ends and being slidably disposed within the inner lumen (16) and the inner lumen (16) having a length between the proximal guidewire port (17) and the proximal perfusion ports (21) which is adapted to hold the distal end of the guidewire (20, 23) therein so as to not interfere with blood flow within the inner lumen (16) between the proximal (17) and distal (18) perfusion ports.

12. The catheter (10) of any one of the preceding claims further characterized by the guidewire (20, 23) being slidably disposed within the inner lumen (16) to facilitate advancement of the catheter (10) thereover.

## Patentansprüche

1. Gefäßkatheter (10) umfassend einen Körper (11) mit einem distalen und einem proximalen Ende und einem aufweitbaren Element (12) am distalen Ende; ein Innenlumen (16); eine proximale und eine distale Führungsdrahtöffnung (17; 18); und Perfusionsöffnungen (21, 22) in der Wand des Katheterkörpers (11), wobei die Perfusionsöffnungen (21, 22) zwischen der proximalen Führungsdrahtöffnung (17) und der distalen Führungsdrahtöffnung (18) im distalen Ende des Katheters (10) mit dem Innenlumen (16) in Fluidverbindung stehen, die Führungsdrahtöffnung (17) proximal sowohl zu dem aufweitbaren Element (12) als auch zu den proximalen Perfusionsöffnungen (21) und distal zu dem proximalen Ende des Katheters angeordnet ist, das distale Ende des Katheterkörpers (11) einen ersten, zu dem aufweitbaren Element (12) proximal gelegenen Abschnitt aufweist, der Perfusionsöffnungen besitzt, und einen zweiten Abschnitt, der proximal gelegen ist zu dem ersten Abschnitt, der distal gelegen ist zu der proximalen Führungsdrahtöffnung (17), und der keine Perfusionsöffnungen besitzt, wobei der Katheter (10) über einen Führungsdraht (23) vorgeschoben werden kann, der verschieblich im Innenlumen (16) angeordnet ist, wobei das aufweitbare Element (12) an dem Katheter (10) so aufgeweitet werden kann, daß es an dieser Stelle ein Blutgefäß wenigstens teilweise verschließt, so daß Blut durch die proximalen Perfusionsöffnungen (21) und das Innenlumen (16) und aus den distalen Perfusionsöffnungen (22) hinaus fließt, wobei das aufweitbare Element (12) kontrahierbar ist, damit der Katheter (10) leichter aus dem Patienten entfernt werden kann, wobei der Katheter dadurch gekennzeichnet ist, daß:
der erste Abschnitt eine maximale äußere Querabmessung besitzt, die größer ist als die maximale äußere Querabmessung des zweiten Abschnitts;
das Innenlumen (16) im distalen Abschnitt des Katheterkörpers (11) zwischen der distalen Führungsdrahtöffnung (18) und der proximalen Führungsdrahtöffnung (17) und 10 bis 50 cm proximal von der distalen Führungsdrahtöffnung (18) verläuft.

2. Katheter nach dem vorhergehenden Anspruch 1, des weiteren dadurch gekennzeichnet, daß:
die proximalen Perfusionsöffnungen (21) entlang der Länge des ersten Abschnitts des distalen Endes des Katheterkörpers (11) verlaufen;
eine Einrichtung (26, 27) vorgesehen ist, die einen Abschnitt des Katheterkörpers (11) proximal zu der proximalen Führungsdrahtöffnung (17) versteift, damit sich der Katheter (10) besser schieben läßt;
die proximalen Perfusionsöffnungen (21) zwischen der in dem Katheterkörper (11) vorgesehenen proximalen Führungsdrahtöffnung (17) und dem aufweitbaren Element (12) angeordnet sind;
die in dem Katheterkörper (11) vorgesehenen distalen Perfusionsöffnungen (22) zwischen der distalen Führungsdrahtöffnung (18) und dem distalen Ende des aufweitbaren Elements (12) angeordnet sind;
die proximale Führungsdrahtöffnung (17) mindestens 10 cm, aber nicht mehr als 50 cm, vom distalen Ende des Katheters (10) angeordnet ist, so daß der Katheter (10) ein leicht auswechselbarer Dilatationskatheter (10) ist.

3. Katheter (10) nach Anspruch 2, des weiteren dadurch gekennzeichnet, daß die Einrichtung (26, 27) zum Versteifen des Katheterkörpers (11) proximal zu der proximalen Führungsdrahtöffnung (17) angeordnet ist und ein Stab (26) ist, der fest in einem Innenlumen (27) des Katheterkörpers sitzt, das sich von dem proximalen Ende des Katheterkörpers (11) zu einer Stelle im Bereich der proximalen Führungsdrahtöffnung (17) erstreckt.

4. Katheter (10) nach einem der vorhergehenden Ansprüche, des weiteren dadurch gekennzeichnet, daß das aufweitbare Element (12) ein aufblasbarer, relativ unelastischer Ballon (12) ist, der sich zum Aufweiten einer Stenose eignet, wobei der Ballon (12) aus einem Kunstharz besteht, das ausgewählt ist aus der Gruppe umfassend Polyethylen und Polyethylenterephthalat.

5. Katheter (10) nach einem der vorhergehenden Ansprüche, des weiteren dadurch gekennzeichnet, daß der Katheter (10) ein Aufblaslumen (13) besitzt, das distal vom proximalen Ende des Katheterkörpers (11) bis zum Inneren des Ballons (12) verläuft, wobei die proximale Führungsdrahtöffnung (17) etwa 12 bis etwa 40 cm von der distalen Führungsdrahtöffnung (18) angeordnet ist.

6. Katheter (10) nach einem der vorhergehenden Ansprüche, des weiteren dadurch gekennzeichnet, daß das Aufblaslumen (13) einen D-förmigen Querschnitt besitzt und ein dünner Draht (32) in dem D-förmigen Aufblaslumen (13) verläuft, um zu verhindern, daß Luftblasen in einer Ecke des Aufblaslumens hängenbleiben.

7. Katheter (10) nach einem der vorhergehenden Ansprüche, des weiteren gekennzeichnet durch einen in der Wand des Katheterkörpers (11) vorgesehenen Schlitz (31), der wenigstens zum Teil das Innenlumen (16) begrenzt, das von der proximalen Führungsdrahtöffnung (17) zu einer Stelle im Bereich der proximalen Perfusionsöffnungen (21) verläuft.

8. Katheter (10) nach Anspruch 6, des weiteren dadurch gekennzeichnet, daß es etwa 6 bis etwa 20 proximale Perfusionsöffnungen (21) und etwa 4 bis etwa 12 distale Perfusionsöffnungen (22) in der Katheterwand gibt.

9. Katheter (10) nach einem der vorhergehenden Ansprüche, des weiteren dadurch gekennzeichnet, daß der Führungsdraht (20, 23) und der Katheter (10) so dimensioniert sind, daß man den Führungsdraht (20, 23) wenigstens teilweise aus dem Abschnitt des Innenlumens (16) zwischen den proximalen (21) und den distalen (22) Perfusionsöffnungen herausziehen kann.

10. Katheter (10) nach einem der vorhergehenden Ansprüche, des weiteren gekennzeichnet durch einen zweiten Dilatationskatheter als Ersatz bzw. zur Verwendung als weiteren Katheter zu dem Katheter (10), wobei der zweite Dilatationskatheter ebenfalls ein aufweitbares Element (12) an seinem distalen Ende und darin ein Innenlumen (16) besitzt, das in einem distalen Abschnitt des Katheterkörpers zwischen einer distalen Führungsdrahtöffnung und einer proximalen Führungsdrahtöffnung etwa 10 bis etwa 50 cm proximal von der distalen Führungsdrahtöffnung verläuft, sowie Perfusionsöffnungen in der Wand des Katheterkörpers, die zwischen der proximalen Führungsdrahtöffnung und der distalen Führungsdrahtöffnung im distalen Ende des Katheterkörpers mit dem Innenlumen (16) in Fluidverbindung stehen; wobei der zweite Dilatationskatheter auf dem Führungsdraht (20, 23) befestigt wird, indem man das proximale Ende des Führungsdrahtes (20, 23) durch das Lumen schiebt, bis ein Abschnitt desselben aus der proximalen Führungsdrahtöffnung ragt; indem man dann den aus der proximalen Öffnung des Katheters ragenden Abschnitt des Führungsdrahtes (20, 23) festhält; und indem man den Katheter über den Führungsdraht (20, 23) in und durch das Gefäßsystem des Patienten schiebt, bis der Katheter an einer gewünschten Stelle darin positioniert ist.

11. Katheter (10) nach einem der vorhergehenden Ansprüche, des weiteren dadurch gekennzeichnet, daß der Führungsdraht (20, 23) ein proximales und ein distales Ende besitzt und verschieblich in dem Innenlumen (16) angeordnet ist, und das Innenlumen (16) eine Länge zwischen der proximalen Führungsdrahtöffnung (17) und den proximalen Perfusionsöffnungen (21) besitzt, die geeignet ist, das distale Ende des Führungsdrahtes (20, 23) darin zu halten, so daß der Blutstrom in dem Innenlumen (16) zwischen den proximalen (21) und den distalen (22) Perfusionsöffnungen nicht beeinträchtigt wird.

12. Katheter (10) nach einem der vorhergehenden Ansprüche, des weiteren dadurch gekennzeichnet, daß der Führungsdraht (20, 23) verschieblich in dem Innenlumen (16) gelagert ist, damit der Katheter (10) leichter darüber vorgeschoben werden kann.

## Revendications

1. Cathéter vasculaire (10) comprenant un corps (11) présentant des extrémités distale et proximale et un élément dilatable (12) disposé sur l'extrémité distale, une lumière intérieure (16), un orifice proximal de fil de guidage et un orifice distal de fil de guidage (17, 18), et des orifices de transfert (21, 22) ménagés dans la paroi du corps de cathéter (11), les orifices de transfert (21, 22) communiquant avec une possibilité de passage de fluide avec la lumière intérieure (16) entre l'orifice proximal de fil de guidage (17) et l'orifice distal de fil de guidage (18) dans l'extrémité distale du cathéter (10), l'orifice de fil de guidage (17) étant disposé d'une manière proximale par rapport à la fois à l'élément dilatable (12) et aux orifices proximaux de transfert (21) et d'une manière distale par rapport à l'extrémité proximale du cathéter, l'extrémité distale du corps de cathéter (11) formant une première partie proximale par rapport à l'élément dilatable (12) qui comporte des orifices de transfert et une seconde partie proximale par rapport à la première partie et distale par rapport à l'orifice proximal de fil de guidage (17) et qui ne comporte pas d'orifices de transfert, le cathéter (10) pouvant être avancé par-dessus le fil de guidage (23) disposé d'une manière coulissante dans la lumière intérieure (16), l'élément dilatable (12) situé sur le cathéter (10) étant dilatable de façon obturer au moins partiellement un vaisseau sanguin en un emplacement obligeant le sang à traverser les orifices proximaux de transfert (21) et la lumière intérieure (16) et à sortir des orifices distaux de transfert (22), l'élément dilatable (12) pouvant être contracté de façon à faciliter un retrait du cathéter (10) hors du patient, le cathéter étant caractérisé par :
la première partie ayant une dimension transversale extérieure maximale qui est supérieure à la dimension transversale extérieure maximale de la seconde partie,
la lumière intérieure (16) s'étendant dans la partie distale du corps de cathéter (11) entre l'orifice distal de fil de guidage (18) et l'orifice proximal de fil de guidage (17) et sur 10 à 50 cm de manière proximale à partir de l'orifice distal de fil de guidage (18).

2. Cathéter suivant la revendication 1 précédente, caractérisé en outre par :
les orifices proximaux de transfert (21) s'étendant sur une certaine longueur de la première partie de l'extrémité distale du corps de cathéter (11),
des moyens (26, 27) pour raidir une partie du corps de cathéter (11) de manière proximale par rapport à l'orifice proximal de fil de guidage (17) afin de conférer au cathéter (10) une meilleure aptitude à être poussé,
les orifices proximaux de transfert (21) étant situés entre l'orifice proximal de fil de guidage (17) ménagé dans le corps de cathéter (11) et l'élément dilatable (12),
les orifices distaux de transfert (22) ménagés dans le corps de cathéter (11) étant situés entre l'orifice distal de fil de guidage (18) et l'extrémité distale de l'élément dilatable (12),
l'orifice proximal de fil de guidage (17) étant disposé à au moins 10 cm, mais pas plus de 50 cm à partir de l'extrémité distale du cathéter (10), de sorte que le cathéter (10) est un cathéter de dilatation (10) facilement échangeable.

3. Cathéter (10) de la revendication 2, caractérisé en outre en ce que les moyens (26, 27) servant à raidir le corps de cathéter (11) sont situés d'une manière proximale par rapport à l'orifice proximal de fil de guidage (17) et consistent en une tige (26) engagée d'une manière serrée dans une lumière intérieure (27) du corps de cathéter et s'étendant de l'extrémité proximale du corps de cathéter (11) jusqu'en un emplacement proximal par rapport à l'orifice proximal de fil de guidage (17).

4. Cathéter (10) de l'une quelconque des revendications précédentes, caractérisé en outre en ce que l'élément dilatable (12) est un ballonnet (12) gonflable et relativement non élastique convenant pour dilater une sténose, le ballonnet (12) étant formé d'une résine plastique choisie dans le groupe constitué du polyéthylène et du téréphtalate de polyéthylène.

5. Cathéter (10) de l'une quelconque des revendications précédentes, caractérisé en outre en ce que le cathéter (10) comporte une lumière de gonflage (13) qui s'étend d'une manière distale de l'extrémité proximale du corps de cathéter (11) jusqu'à l'intérieur du ballonnet (12), l'orifice proximal de fil de guidage (17) étant disposé d'environ 12 à environ 40 cm à partir de l'orifice distal de fil de guidage (18).

6. Cathéter (10) de l'une quelconque des revendications précédentes, caractérisé en outre en ce que la lumière de gonflage (13) présente une section transversale en D et un fil mince (32) s'étend dans la lumière de gonflage (13) de façon à empêcher la retenue de bulles d'air dans un coin de la lumière de gonflage.

7. Cathéter (10) de l'une quelconque des revendications précédentes, caractérisé en outre par une fente (31), ménagée dans la paroi du corps de cathéter (11) définissant au moins en partie la lumière intérieure (16), qui s'étend de l'orifice proximal de fil de guidage (17) jusqu'en un emplacement proximal par rapport aux orifices proximaux de transfert (21).

8. Cathéter (10) de la revendication 6, caractérisé en outre en ce qu'il existe environ 6 à environ 20 orifices proximaux de transfert (21) et environ 4 à environ 12 orifices distaux de perfusion (22) ménagés dans la paroi de cathéter.

9. Cathéter (10) de l'une quelconque des revendications précédentes, caractérisé en outre en ce que le fil de guidage (20, 23) et le cathéter (10) sont dimensionnés de façon à permettre que l'on retire au moins partiellement le fil de guidage (20, 23) de la partie de la lumière intérieure (16) entre les orifices proximal (17) et distal (18) de transfert.

10. Cathéter (10) de l'une quelconque des revendications précédentes, caractérisé en outre par un second cathéter de dilatation destiné à remplacer le cathéter (10) ou à être utilisé en tant que cathéter supplémentaire par rapport à celui-ci, le second cathéter de dilatation comportant également un élément dilatable (12) sur son extrémité distale et, intérieurement, une lumière intérieure (16) s'étendant dans une partie distale du corps de cathéter entre un orifice distal de fil de guidage et un orifice proximal de fil de guidage sur environ 10 à environ 50 cm de manière proximale à partir de l'orifice distal de fil de guidage, et des orifices de transfert ménagés dans la paroi du corps de cathéter et communiquant avec une possibilité de passage de fluide avec la lumière intérieure (16) entre l'orifice proximal de fil de guidage et l'orifice distal de fil de guidage dans l'extrémité distale du corps de cathéter, le second cathéter de dilatation étant monté sur le fil de guidage (20, 23) en faisant passer l'extrémité proximale du fil de guidage (20, 23) dans la lumière jusqu'à ce qu'une partie de celui-ci s'étende hors de l'orifice proximal de fil de guidage, en maintenant la partie du fil de guidage (20, 23) s'étendant hors de l'orifice proximal du cathéter, et en faisant avancer le cathéter par-dessus le fil de guidage (20, 23) dans et à travers le système vasculaire du patient jusqu'à ce que le cathéter soit positionné en un emplacement voulu dans celui-ci.

11. Cathéter (10) de l'une quelconque des revendications précédentes, caractérisé en outre en ce que le fil de guidage (20, 23) présente des extrémités proximale et distale et est disposé d'une manière coulissante dans la lumière intérieure (16) et la lumière intérieure (16) présente une longueur entre l'orifice proximal de fil de guidage (17) et les orifices proximaux de transfert (21) qui permet de maintenir l'extrémité distale du fil de guidage (20, 23) dans celle-ci de manière à ne pas faire obstacle à un écoulement de sang dans la lumière intérieure (16) entre les orifices proximal (17) et distal (18) de transfert.

12. Cathéter (10) de l'une quelconque des revendications précédentes, caractérisé en outre en ce que le fil de guidage (20, 23) est disposé d'une manière coulissante dans la lumière intérieure (16) de façon à faciliter un mouvement d'avance du cathéter (10) par-dessus celui-ci.
